# EUROPEAN PATENT SPECIFICATION

(11) **EP 3 083 660 B1**
(45) Date of publication and mention of the grant of the patent: **12.02.2020**
(21) Application number: 14830896.8
(22) Date of filing: 19.12.2014
(51) Int. Cl.: C07K 14/11, A61K 39/145

(54) **METHOD OF INFLUENZA ANTIGEN PRODUCTION**
VERFAHREN ZUR HERSTELLUNG EINES INFLUENZA ANTIGENS
PROCÉDÉ DE PRODUCTION D'UN ANTIGÈNE DE LA GRIPPE

(30) Priority: 20.12.2013 PL 40663113
(43) Date of publication of application: 26.10.2016
(73) Proprietor: Instytut Biochemii i Biofizyki PAN, 02-106 Warszawa (PL); Instytut Biotechnologii i Antyiotyków, 02-516 Warszawa (PL); Uniwersytet Gdanski, 80-309 Gdansk (PL)
(72) Inventor: KOPERA, Edyta, PL-39-300 Mielec (PL); GRZELAK, Krystyna Maria, PL-01-820 Warszawa (PL); ZAGÓRSKI-OSTOJA, Wlodzimierz Wiktor, PL-02-665 Warszawa (PL); SZEWCZYK, Boguslaw, PL-80-156 Gdansk (PL); SACZYNSKA, Violetta Teresa, PL-01-494 Warszawa (PL); FLORYS, Katarzyna Natalia, PL-27-570 Iwaniska (PL); CECUDA-ADAMCZEWSKA, Violetta, PL-03-468 Warszawa (PL); MACIOLA, Agnieszka Katarzyna, Rybnik 44-217 (PL); PIETRZAK, Maria Anna, Otwock 05-400 (PL); ZDANOWSKI, Konrad, Siedlce 08-110 (PL)
(74) Representative: Twardowska, Aleksandra
(86) International application number: PCT/PL2014/000148
(87) International publication number: WO 2015/093996

(56) References cited:
- WO-A1-2012/011868
- WO-A2-2007/022425
- WO-A2-2013/043067
- US-A1- 2007 286 873
- SAELENS XAVIER ET AL: "Protection of mice against a lethal influenza virus challenge after immunization with yeast-derived secreted influenza virus hemagglutinin", EUROPEAN JOURNAL OF BIOCHEMISTRY, WILEY-BLACKWELL PUBLISHING LTD, GB, vol. 260, no. 1, 1 February 1999 (1999-02-01), pages 166-175, XP002194212, ISSN: 0014-2956, DOI: 10.1046/J.1432-1327.1999.00150.X cited in the application
- SHOJI Y ET AL: "Plant-derived hemagglutinin protects ferrets against challenge infection with the A/Indonesia/05/05 strain of avian influenza", VACCINE, ELSEVIER LTD, GB, vol. 27, no. 7, 11 February 2009 (2009-02-11), pages 1087-1092, XP026080004, ISSN: 0264-410X, DOI: 10.1016/J.VACCINE.2008.11.108 [retrieved on 2009-02-11]
- R. P. DE VRIES ET AL: "Glycan-Dependent Immunogenicity of Recombinant Soluble Trimeric Hemagglutinin", JOURNAL OF VIROLOGY, vol. 86, no. 21, 22 August 2012 (2012-08-22), pages 11735-11744, XP055111254, ISSN: 0022-538X, DOI: 10.1128/JVI.01084-12
- CREGG J M ET AL: "RECOMBINANT PROTEIN EXPRESSION IN PICHIA PASTORIS", MOLECULAR BIOTECHNOLOGY, HUMANA PRESS, INC, US, vol. 16, no. 1, 1 September 2000 (2000-09-01), pages 23-52, XP001078868, ISSN: 1073-6085, DOI: 10.1385/MB:16:1:23
- SHIRA ALBECK ET AL: "Three-dimensional structure determination of proteins related to human health in their functional context at The Israel Structural Proteomics Center (ISPC)", ACTA CRYSTALLOGRAPHICA SECTION D BIOLOGICAL CRYSTALLOGRAPHY, vol. 61, no. 10, 28 September 2005 (2005-09-28), pages 1364-1372, XP055181123, ISSN: 0907-4449, DOI: 10.1107/S0907444905023565
- SUBATHRA MURUGAN ET AL: "Recombinant haemagglutinin protein of highly pathogenic avian influenza A (H5N1) virus expressed in Pichia pastoris elicits a neutralizing antibody response in mice", JOURNAL OF VIROLOGICAL METHODS, vol. 187, no. 1, 1 January 2013 (2013-01-01), pages 20-25, XP055180947, ISSN: 0166-0934, DOI: 10.1016/j.jviromet.2012.07.026
- WANG SHIXIA ET AL: "Hemagglutinin (HA) proteins from H1 and H3 serotypes of influenza A viruses require different antigen designs for the induction of optimal protective antibody responses as studied by codon-optimized HA DNA vaccines", JOURNAL OF VIROLOGY, THE AMERICAN SOCIETY FOR MICROBIOLOGY, US, vol. 80, no. 23, 1 December 2006 (2006-12-01), pages 11628-11637, XP002455308, ISSN: 0022-538X, DOI: 10.1128/JVI.01065-06
- Edyta Kopera ET AL: "Expression, purification and characterization of glycosylated influenza H5N1 hemagglutinin produced in Pichia pastoris", Acta biochimica Polonica, 1 January 2014 (2014-01-01), page 597, XP055180772, Poland Retrieved from the Internet: URL:http://www.ncbi.nlm.nih.gov/pubmed/252 10934
- VEIT MICHAEL ET AL: "Association of influenza virus proteins with membrane rafts.", ADVANCES IN VIROLOGY 2011, vol. 2011, 370606, 25 July 2011 (2011-07-25), ISSN: 1687-8647

### SEQUENCE LISTING

<110> Instytut Biochemii i Biofizyki PAN Instytut Biotechnologii i Antybiotyków
<120> The antigen, the influenza vaccine, the system for vaccine manufacturing, the method of antigen production, and use of the antigen to produce an influenza vaccine
<130> 24861/PCT/14
<140> PCT/PL2014/
   <141> 2014-12-18
<150> PL406631
   <151> 2013-12-20
<160> 18
<170> PatentIn version 3.5
<210> 1
   <211> 33
   <212> DNA
   <213> Artificial Sequence
<220>
   <223> primer 1 used in first stage of the PCR
<400> 1
   catcatcatc atgatcagat ttgcattggt tac 33
<210> 2
   <211> 29
   <212> DNA
   <213> Artificial Sequence
<220>
   <223> primer 2 used in the first stage in PCR in example 1
<400> 2
   ccttggatgg ttactcgccg gcgatagcg 29
<210> 3
   <211> 37
   <212> DNA
   <213> Artificial Sequence
<220>
   <223> primer 3 used in the second stage of PCR
<400> 3
   gcacgatcga tgcatcatca tcatcatcat gatcaga 37
<210> 4
   <211> 29
   <212> DNA
   <213> Artificial Sequence
<220>
   <223> primer 4 used in second stage of PCR in example 1
<400> 4
   ccttggatgg ttactcgccg gcgatagcg 29
<210> 5
   <211> 51
   <212> DNA
   <213> Artificial Sequence
<220>
   <223> CDNA of 1569 bp was amplified in PCR reaction with two primers - primer 5 and primer 6 (example 8)
<400> 5
   tccgtggaat tccatcatca tcatcatcat gacacattat gtataggtta t 51
<210> 6
   <211> 33
   <212> DNA
   <213> Artificial Sequence
<220>
   <223> CDNA of 1569 bp was amplified in PCR reaction with two primers - primer 5 and primer 6 (example 8)
<400> 6
   tcaataccgc ggtcaaatga actggcgaca gtt 33
<210> 7
   <211> 43
   <212> DNA
   <213> Artificial Sequence
<220>
   <223> The cleavage site (RRRKKR) in the hemagglutinin sequence of influenza virus H5N1 strain (A/swan/Poland/305-135V08/2006(H5N1)) was deleted in a two step site-directed mutagenesis reaction using the primers 7 to 10 (example 8)
<400> 7
   gccctcaagg agagaaaaag agaggactat ttggagctat agc 43
<210> 8
   <211> 43
   <212> DNA
   <213> Artificial Sequence
<220>
   <223> The cleavage site (RRRKKR) in the hemagglutinin sequence of influenza virus H5N1 strain (A/swan/Poland/305-135V08/2006(H5N1)) was deleted in a two step site-directed mutagenesis reaction using the primers 7 to 10 (example 8)
<400> 8
   cgggagttcc tctctttttc tctcctgata aacctcgata tcg 43
<210> 9
   <211> 34
   <212> DNA
   <213> Artificial Sequence
<220>
   <223> The cleavage site (RRRKKR) in the hemagglutinin sequence of influenza virus H5N1 strain (A/swan/Poland/305-135V08/2006(H5N1)) was deleted in a two step site-directed mutagenesis reaction using the primers 7 to 10 (example 8)
<400> 9
   gccctcaagg agagggacta tttggagcta tagc 34
<210> 10
   <211> 34
   <212> DNA
   <213> Artificial Sequence
<220>
   <223> The cleavage site (RRRKKR) in the hemagglutinin sequence of influenza virus H5N1 strain (A/swan/Poland/305-135V08/2006(H5N1)) was deleted in a two step site-directed mutagenesis reaction using the primers 7 to 10 (example 8)
<400> 10
   cgggagttcc tctccctgat aaacctcgat atcg 34
<210> 11
   <211> 33
   <212> DNA
   <213> Artificial Sequence
<220>
   <223> The CDNA (H5HAdel) of 1527 bp was amplified in a two step PCR using in the first stage primers 11 and 12 (example 8)
<400> 11
   gcacgatcga tggatcagat ttgcattggt tac 33
<210> 12
   <211> 42
   <212> DNA
   <213> Artificial Sequence
<220>
   <223> The CDNA (H5HAdel) of 1527 bp was amplified in a two step PCR using the in the first stage primers 11 and 12 (example 8)
<400> 12
   agttatcctt ggatggttgt agtagtaact ggcgccatag cg 42
<210> 13
   <211> 33
   <212> DNA
   <213> Artificial Sequence
<220>
   <223> The cDNA (H5HAdel) of 1527 bp was amplified in a two step PCR using in the second stage primers 13 and 14 (example 8)
<400> 13
   gcacgatcga tggatcagat ttgcattggt tac 33
<210> 14
   <211> 24
   <212> DNA
   <213> Artificial Sequence
<220>
   <223> The cDNA (H5HAdel) of 1527 bp was amplified in a two step PCR using in the second stage primers 13 and 14 (example 8)
<400> 14
   gtagtagtaa ctggcgccat agcg 24
<210> 15
   <211> 27
   <212> DNA
   <213> Artificial Sequence
<220>
   <223> The CDNA fragment (H3) encoding the hydrophilic domain of the hemagglutinin of influenza H3N2 virus was amplified by PCR using primers 15 and 16 (example 8)
<400> 15
   tccgtgggta ccgcaacgct gtgcctt 27
<210> 16
   <211> 30
   <212> DNA
   <213> Artificial Sequence
<220>
   <223> The cDNA fragment (H3) encoding the hydrophilic domain of the hemagglutinin of influenza H3N2 virus was amplified by PCR using primers 15 and 16 (example 8)
<400> 16
   gctagcggcc gctcaatctt tgtaccctga 30
<210> 17
   <211> 30
   <212> DNA
   <213> Artificial Sequence
<220>
   <223> The cDNA fragment (H7) encoding the hydrophilic domain of the hemagglutinin of influenza H7N9 virus was amplified by PCR using primers 17 and 18 (example 8)
<400> 17
   tccgtgggta ccgacaaaat ctgcctcgga 30
<210> 18
   <211> 30
   <212> DNA
   <213> Artificial sequence
<220>
   <223> The cDNA fragment (H7) encoding the hydrophilic domain of the hemagglutinin of influenza H7N9 virus was amplified by PCR using primers 17 and 18 (example 8)
<400> 18
   gctagcggcc gctcaatctt tgtagccgct 30

## Description

The subjects of an invention are the method for producing a secreted ectodomain of influenza virus haemagglutinin (HA) without signal peptide and the method for producing an influenza virus vaccine. The invention involves a method of production of vaccine antigen. The outcome as stated in the invention leads to obtaining a highly immunogenic antigen, which does not require the contact with the virus but just with haemagglutinin gene. The vaccine obtained using the method of the invention does not contain the virus or its parts, but only pure antigen.

Influenza is an infectious viral disease affecting both humans and animals worldwide. Influenza epidemics occur each year, causing high morbidity and mortality. It is caused by a polymorphic RNA virus of the Orthomyxoviridae family, with 125 nanometres of diameter of each particle. There are three main types of influenza marked with letters A, B, and C respectively. The influenza A viruses can be subdivided into serotypes based on two surface glycoprotein: haemagglutinin (HA) and neuraminidase (NA). So far 16 HA subtypes and 9 NA (N1-N9) subtypes have been identified. The flocks of wild aquatic birds are natural hosts of all the influenza virus subtypes. Only three HA subtypes (H1, H2, and H3) and two NA subtypes (N1 and N2) have been constanly prevalent in the human population since 1918. However, it needs to be emphasized that H5N1 virus, the 'bird flu' virus which appeared in the 90s of the 20th century can also infect people. A relevant characteristic of the influenza virus is its capacity to change constantly, which is caused by mutations in the genes encoding proteins HA and NA [Wiley D and Skehel J. 1987]. Those changes are usually activated by two mechanisms: antigenic shift and antigenic drift. Inaccurate RNA polymerase results in high proofreading mechanisms error, which in turn leads to the change of amino acids in HA sequence. This mechanism is called antigenic drift. If, in turn, the organism is infected with two different strains of virus simultaneously, RNA segments will be re-assorted, which will lead to antigenic shift. This is usually connected with a new type of virus being formed. Both antigenic drift and antigenic shift are impossible to predict and may cause the formation of a highly pathogenic virus. Vaccination remains the most effective method of protection against influenza virus and the way to reduce the risk of epidemic or pandemic spread. The active substance in the influenza vaccine is the whole inactive and purified virion or its parts. The vaccines of this type available in the market protect 60%-90% of adults and 23%-72% of the elderly. The process of obtaining the vaccine includes the following stages: (1) Isolating the influenza virus (2) Genetic and antigenic characteristics of the isolated virus (3) Selecting the strain of virus in the coming season (4) Preparing the most virulent strain in the coming season through reassortment (5) Making the virulent strain available to the manufacturers of the vaccine (6) Estimating the value of the obtained strain of virus for vaccine manufacturing (7) Virus manufacturing. The time required to produce the vaccine is from 7 to 8 months. Stage (4) i.e. preparing the most virulent strain of virus through reassortment requires the use of genome segments from the virus strain effectively multiplying in embryonated hen eggs. The most common strain is A/PR/8/34. Thus, the obtained vaccine strain is different from the virus strain isolated for a particural season. It is common knowledge that influenza viruses isolated from the infected organisms do not multiply well in embryonated hen eggs or cell lines. It needs to be emphasized that the influenza vaccine manufactured in a traditional way and based on multiplying of the virus in hen emryos, though well developed, has its fundamental drawbacks and limitations. Except the above mentioned lack of full homology between the virus isolated from the environment and the one the manufacturer is provided with, an important downside of this method is its long-drawn process. It has many stages as well as high manufacturing costs as it is connected with the breeding of thousands of embryonated hen eggs. It is worth pointing out that two eggs on average are required for producing one vaccine dose. However, the most important aspect in the process of vaccine production is its safety. An increasing number of patients reacting with strong allergy to protein contamination in the vaccines produced in the traditional method is now being observed. Alternative methods are now being explored. One of them is vaccine manufacturing with the use of cell lines. Despite extensive research carried out to develop the method, it hasn't been widely used. FDA alowed a few cell lineages to be used in vaccine production, for example Vero cells or MDCK. However, due to the potentiality of cancerogenic agents and protein contamination, the vaccines produced from the cell lines have never gone beyond the research phase. As the antibodies neutralising the influenza virus are directed mainly against the HA protein, a dynamic development in subunit vaccine research is currently being observed. This type of vaccine contains recombinant HA protein, obtained by means of genetic engineering with different expression systems. Haemagglutinin is a glycoprotein most prolifically found on the surface of the virus. It has two main functions in the process of infection: it enables binding the virus to host cells and, in the endocytosis, it is involved in mediating in the fusion of the virus with endosome membrane. Haemagglutinin occurs on the surface of the virus in homotrimeric form. Each monomer consists of two subunits: HA1 and HA2, linked by a disulphide bond, which constitutes a hydrophilic domain. A monomer molecule is synthesized as inactive precursor HA0. The protein undergoes N-linked glycosylation and its final molecular weight is approximately 80 kDA. Cleavage of the precursor HA0 protein into two subunits is the prerequisite for the virus infectivity. Haemagglutinin of low pathogenic influenza viruses at a specific cleavage site has a single arginin residue and is activated by trypsin proteases. With high pathogenic viruses, multiplication of basic amino acids at cleavage site of the HA0 protein is present, therefore it is recognized by a series of host proteases. Due to this change, the virus can spread rapidly and replicate in almost every organ, causing a systemic infection. Haemagglutinin is the most important antigen when serological specificity of the virus strain is determined. This protein contains a lot of epitopes located mainly within HA1 subunit. The presence of neutralizing IgG and IgA antobodies, specific for influenza virus haemagglutinin, is linked with the body's immunity to the infection and disease [Clements, 1992]. One of the systems used for recombinant influenza vaccine manufacturing is baculovirus expression system based on insect cells. The method of obtaining haemagglutinin in baculovirus system is the object of US 5858368 patent. However, this solution involves high manufacturing costs connected with the demand for high qualified staff and expensive reagents. Therefore, new, inexpensive and safe methods of producing vaccine antigens are still being explored. Growing expectations for high immunogenic antigens are involved with expression system based on yeast cells. Patent No US4752473 is concerned with haemagglutinin expression in Saccharomyces cereviciae. This system enables effective HA manufacturing. However, an obstacle to using this strategy is high protein glycosylation. Virus H10N7 haemagglutinin was obtained in Schizosaccharomces pombe cells (Wu et al, 2009) whereas H5N2 virus in Kluyveromyces lactis yeast (Su-Ming Tsai et al., 2011). Saelens et al. in their research in 1999 obtained HA of H3N2 virus in Pichia cells of GS115 strain. cDNA of HA without C-terminal transmembrane domain was cloned into the pPIG9 vector containing a α-factor sequence. A secreted protein had a form of a monomer. Trimeric forms have not been documented. The protein was not cleaved into subunits. Xu Ym et al. (2006) in their paper published in a Chinese journal have reported H5, H7, and H9 virus HA expression in Pichia cells of GS115 strain. pPIG9 vector was used. Chi Y. Wang et al. (2007) obtained H5N2 virus haemagglutinin in P. pastoris cells of GS115 strain. This haemagglutinin was cleaved into subunits. A full length HA gene was cloned into pPICZαA vector. A protein partly secreted into the culture medium and mostly remaining in the cell was obtained. Subatha et al. obtained a full length HA of H5N1 virus in P.pastoris system; however, only intracellularly. pPICZ C vector was used. Yang K. et al. obtained only rHA1 domain of H5N1 virus. Shehata et al. also obtained only HA1 subunit of H5N1 virus in Pichia cells of GS115 and SMD1168H strains, using the vector pGAPZαC which contained His-Tag. Athamaran TN. et al. (2011) and Athamaran et al. (2012) obtained a full length HA (HA0) of H1N1 virus in GS115 P. Pastoris strain. pPICK9K vector with α-factor was used. HA0 (80kDA) that was produced extracellularly was not cleaved and appeared in a trimeric form (approx. 240kDa) as well.

Despite the existing solutions that have emerged so far, there is a constant need to develop a new method of vaccine antigen and new antigen production. All the existing solutions based on multiplying virus in the embryonated eggs involve the need to work with the virus. Along with a multistage production, there are high manufacturing costs that the breeding of thousands of embryonated eggs generates. Fast replicating virus is prepared through genome segment reassortment from the virus strain efficiently replicating in embryonated eggs. An increasing number of people reacting with strong allergy to protein contamination in traditional vaccines is being reported. Moreover, non-attenuated virus can also be present. Cancerous factors and protein contamination may appear in the systems based on cell lines, however, the system is still being researched.
As far as baculovirus system is concerned, reagents are expensive, highly qualified staff is needed, and manufacturing costs are high.

The aim of this invention is to develop a method of manufacturing a vaccine antigen. Although HA protein manufacturing in Pichia cells has been reported, this invention proposes a solution that differs from the published ones in a few respects. The characteristic feature of the antigen manufacturing method is that the antigen is produced extracellularly by Pichia pastoris cells of KM 71 strain (his4, aoxl::ARG4, arg4). It is used for selecting expression vectors containing HIS4 for generating the strains with Mut^{S} phenotype. SMD 1168 (his4, pep4) used for selecting expression vectors containing HIS4 enabling strain selection without the activity of protease A is also described.

It turned out unexpectedly that the solution developed in this invention leads to obtaining a highly immunogenic antigen and does not require the contact with the virus but only haemagglutinin gene. The vaccine does not contain the virus or its parts, but only the purified antigen. The manufacturing method is simple and inexpensive compared to the existing solutions. Furthermore, there is no protein contamination in the vaccine produced in this method. As the virus is mutating rapidly, the vaccine obtained in this way is homologous with the influenza virus strain isolated for a given season. In response to growing demand, it also enables its fast production.

The invention is concerned with a method of manufacturing a vaccine antigen and an antigen, as defined in the claims. The characteristic feature of this method is that (1) the antigen is produced extracellurarly by the Pichia pastoris of KM71 strain. The characteristic trait of the antigen is that (1) it is a protein cleaved at least at one site, wherein one of them is a cleavage site recognized by proteases, connecting HA1 domain with HA2 domain or not cleaved (2), it is a protein linked constantly by a disulphide bond (3) it is a glycosylated protein (4) it appears in trimeric and oligomeric forms (5) it simultaneously appears as a mixture of two domains: HA1 and HA2, the protein linked by a disulphide bond, monomer, and the protein in trimeric and oligomeric form, (6) it is a protein with affinity tag as of His-tag or without an affinity tag at all.

It unexpectedly turned out in the method presented in this invention that Pichia pastoris cells of the KM71 strain produce to the culture medium an HA protein that is: cleaved at least at one site into domain HA1 and HA2, or not cleaved appearing as a protein linked by a disulphide bond and having an oligomeric form, which shows strong immunogenic potential.
The method is a way of obtaining antigen with a variety of applications, including antigen used for WB and ELISA test. It is also useful for vaccine preparation, although it is not limited in its application to vaccine manufacturing. The invention uses the viral strains as defined in the claims. The experts using H5 virus haemagglutinin as an example will point to the possibility of obtaining a highly immunogenic antigen of H1N1, H3N1 etc. influenza viruses.

The subject of invention is the method for producing a secreted ectodomain of influenza virus haemagglutinin (HA) without signal peptide, characterized by the following steps:
a) inserting a sequence encoding the ectodomain of influenza H1 or H5, into a yeast expression vector comprising a signal sequence to direct said protein to the yeast secretory pathway, under control of the alcohol oxidase 1 (AOX1) promoter;
b) introducing said expression vector into Pichia pastoris cells of KM71 strain by electroporation;
c) culturing a Pichia pastoris transformant colony of KM71 strain obtained in step b), wherein the AOX1 promoter is induced with methanol; and
d) isolating the ectodomain of influenza HA without signal peptide from the culture medium of the transformant colony of step c).

Preferably, the secreted ectodomain of HA is a glycosylated protein having a trimeric or oligomeric form.

Preferably, the secreted ectodomain of HA protein comprises a deletion of a cleavage site, wherein 6 amino acids between HA1 and HA2 are removed.

Preferably, the secreted ectodomain of HA is derived from H5 influenza.

Preferably, ein the secreted ectodomain of HA further comprises an affinity tag.

The next subject of invention is the method for producing an influenza virus vaccine comprising a secreted ectodomain of influenza virus HA without signal peptide characterized by
a) obtaining a secreted ectodomain of influenza virus HA without signal peptide as defined above, using the method described above; and
b) manufacturing a vaccine comprising said secreted ectodomain of influenza virus HA without signal peptide.

The attached Figures allow for a better understanding of the essence of the invention.
**Figure 1** shows identification of recombinant protein HA in culture supernatants of KM 71 and SMD 1168 strains. **Figure 1A** shows Western Blot analysis of Anti-His Tag antibody, whereas **Figure 1B** shows Western Blot analysis of Anti-HAl antibody;
**Figure 2** shows the analysis of elution fractions obtained during His₆-HA purification on Ni-NTA Agarose (12% SDS-PAGE, stained with Coomassie), where **Figure 2A** shows KM 71 strain and **Figure 2B** SMD 1168 strain;
**Figure 3** shows the rate of HA protein overexpression in P. pastoris cells of KM 71 strain; (example 3)
**Figure 4** shows the analysis of elution fractions obtained during His₆-HA purification on Ni-NTA Agarose (12% SDS-PAGE, stained with Coomassie) - where **Figure 4A** shows SMD 1168 strain cultured in 28°C, **Figure 4B** shows KM 71 strain cultured in 30°C, **Figure 4C** shows KM 71 strain cultured in 30°C in a presence of Casamino acid; (example 3)
**Figure 5** shows N-glycosylation level of subunit His₆-HA1 after reacting with EndoH.
**Figure 5A** shows Western Blot analysis, anti-HisTag antibodies, whereas **Figure 5B** shows SDS-PAGE analysis;
**Figure 6** shows native gel from example 5;
**Figure 7** shows antibody titre of IgG antibody isotype against H5 in mouse serum after immunization with rHA antigen obtained from Pichia pastoris cells. **Figure 7A** shows various antigen doses, whereas **Figure 7B** shows different routes of antigen application in dose of 25µg; (example 6)
**Figure 8** shows (example 7)
**Figure 9** shows Western blot analysis of various rHAs (H1DHHis₆, H3DH, H5DHdelHis₆, H5DH) overexpression in *P. pastoris* KM 71 and SMD strains. The protein samples were electrophoresed in 12 % SDS-PAGE and transferred onto membrane and identified by anti-His (A, D), anti-H3 (B), and anti-H5 (C) antibody. A, B, C - culture after 72 h of methanol induction (1 ml of culture medium precipitated with TCA). D - culture after 24, 48, 72 and 96 h of methanol induction (1 ml of culture medium precipitated with TCA).
**Figure 10** shows the analysis of elution fractions obtained during HA- His₆ purification on Ni-NTA Agarose (12% SDS-PAGE, stained with Coomassie), where **Figure 10A** shows H1DHHis₆, **Figure 10B** H5DHdelHis₆. **Figure 10C** shows the analysis of elution fraction obtained during H5DH on ion-exchange chromatography (12% SDS-PAGE, stained with Coomassie).
**Figure 11** shows native gel of H1DHHis₆, H5DHdelHis₆ and BSA.
**Figure 12** shows N-glycosylation level of H1DHHis₆ after reacting with EndoH (12% SDS-PAGE, stained with Coomassie).
**Figure 13** shows Superdex-200 gel filtration chromatography of HIDHHis₆ protein purified from *P. pastoris* KM 71 (black line) overlaid with calibration standards (red line). The elution of various protein forms is shown (detection at 280 nm).
**Figure 14** shows transmission electron microscopy - negative staining with uranyl acetate. **Figure 14A** shows the analysis of the individual fractions of the gradient ultracentrifugation of H1DHHis₆ protein (10% DSD-PAGE stained with Coomassie Brilliant Blue R-250). **Figure 14B** Western blot of selected fractions with anti-H1 antibody. **Figure 14C** shows negative stain electron micrograph of H1DHHis₆ protein.
**Figure 14D** shows the analysis of the individual fractions of the gradient ultracentrifugation of H5DHdelHis₆ protein (10% DSD-PAGE stained with Coomassie Brilliant Blue R-250). **Figure 14E** Western blot of selected fractions with anti-H5 antibody. **Figure 14C** shows negative stain electron micrograph of H5DHdelHis₆ protein.
**Figure 15** shows HA specific serum IgG endpoint titers after mice immunization with *P. pastoris* rH1 DHHis₆. The bars represents the average of the titers in sera collected from 10 individuals. The endpoint titer is defined as the highest dilution of serum inducing in ELISA test absorbance 4 times over background. **Figure 15A****.** Mice were injected with various doses of rH1 antigen intradermally (i.d.). Sigma Adjuvant System was used as adjuvant. **Figure 15B****.** Mice were injected with various doses of rH1 antigen subcutaneously. Alhydrogel was used as adjuvant.
**Figure 16** shows HI titers against influenza H5N1/turkey/35/07 virus. H5N1 influenza-specific neutralizing antibodies in chicken immunized with H5DHdelHis₆ protein **(****Figure 16A** **8A****)** and H5DHdel protein **(****Figure 16B** **8B****).**
**Figure 17** shows survival rate of SPF chicken immunized twice with H5DHdelHis₆ protein (black line) and not-immunized SPF chicken (control group, red line) after infection with H5N1/turkey/35/07 virus at 10⁶ EID₅₀.

Herein below are shown exemplary modes of carrying out the invention defined above.

### Example 1. Cloning and overexpression of recombinant protein HA

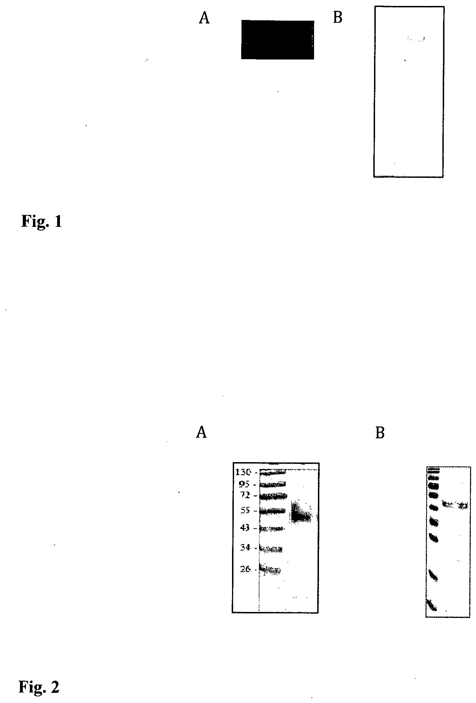

Plasmid pPICZaC (Invitrogen) and plasmid pGEM-Easy/HA carrying H5N1 virus haemagglutinin gene (A/swan/Poland/305-135V08/2006 (H5N1)) have been used for construction of expression vector pPICZaC/HAHis6. Plasmid pPICZaC contains alcohol oxidase promoter 1 (*AOX1*) induced by methanol. The plasmid contains α factor sequence which comes from Saccharomyces cerevisiae, which ensures the secretion of recombinant protein to the medium. cDNA of 1545 bp, which codes hydrophilic domain (17-531aa) of H5N1 virus haemagglutinin (A/swan/Poland/305-135V08/2006 (H5N1)), was amplified in a two-stage reaction PCR, in which the following primers were used:
5'CATCATCATCATGATCAGATTTGCATTGGTTAC-3' and 3'CCTTGGATGGTTACTCGCCGGCGATAGCG-5'. A pair of primers: 5'GCACGATCGATGCATCATCATCATCATCATGATCAGA-3' and 3'CCTTGGATGGTTACTCGCCGGCGATAGCG-5' were used in the second stage. The sequence encoding His-Tag was added to cDNA sequence by the right primer development. PCR prooduct was cloned into pPICZaC expression vector under the control of the AOX promoter. Pichia pastoris cells of strains KM 71 (his4, aox1 ::ARG4, arg4) and SMD 1168 (his4, pep4) (Invitrogen) were transformed with pPICZαC/HAHis₆ plasmid by electroporation. Integration of haemagglutinin gene into P. pastoris genome was confirmed by PCR. KM 71 and SMD 1168 transformant selection was performed using medium containing Zeocin (multiple passaging). A single colony of P. pastoris strain transformed with pPICZαC/HAHis₆ plasmid was cultured in BMGY medium (1% yeast extract, 2% peptone, 100mM potassium phosphate, pH 6.0, 1,34% YNB 0,00004% biotin, 0,5% glycerol). The culture temerature was 28°C with shaking (at the frequency of 160 strokes per minute) until high cell density OD₆₀₀>3 is achieved. After centrifugation the cells were suspended in BMMY medium (1% yeast extract, 2% peptone, 100mM potassium phosphate pH 6.0, 1,34% YNB, 0,00004% biotin, 5% methanol). The presence of recombinant protein both in medium and cells (control), was detected by SDS-PAGE and Western blotting (with anti-HisTag antibody and anti-HA). **Fig. 1** shows the recombinant protein HA identified in culture supernatants of strains: KM71 and SMD1168.

### Example 2. Purification of recombinant HA protein

Recombinant haemagglutinin was purified from culture medium by affinity chromatography, using Ni-NTA Agarose resine. Protein binding to Ni-NTA Agarose was carried out in phosphate buffer solution (PBS) (pH 7.4 and 450 mM NaCl). The binding was carried out for 3 hours at 4°C, constantly shaking. The resine was rinsed with PBS pH 7.4 with 25 mM imidazole. HA protein was eluted from the column with 250 mM imidazole in PBS pH 7.4.

Specific amount of solution from each elution fraction was withdrawn for determining a protein level and SDS-PAGE analysis. The protein amount was determined spectrophotometrically using Bradford method by an absorbance measurement at 595 nm. The protein was then dialyzed against PBS pH 7.4. After dialysis the protein was liophylized and stored in temmperature -20°C. Analysis of elution fractions obtained during His₆-HA purification on Ni-NTA Agarose of KM 71 and SMD 1168 strains was shown in **Fig. 2****.**

### Example 3. Optimization of protein overexpression

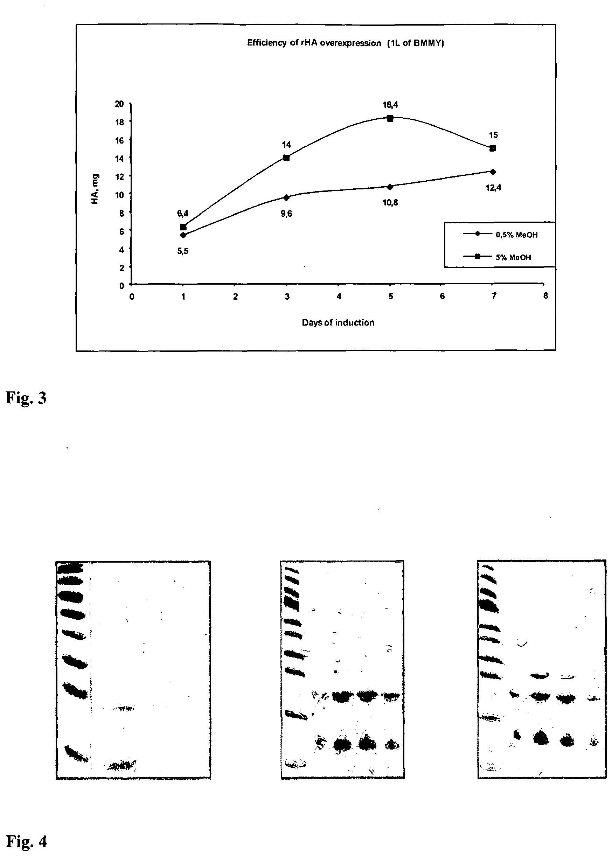

The induction of protein expression was performed from 1 to 7 days. Samples were taken on a daily basis. Recombinant protein was determined by SDS-PAGE and Western blotting tests (with anti-HisTag antibody). Protein expression was optimized in terms of temperature, culture medium composition (i.e. the application of various protease inhibitors), pH or methanol level. The highest expression level was achieved with 5% methanol induction. **Fig. 3** shows overexpression efficiency analysis of HA protein in P.pastoris cells of KM 71 strain.

Protein degradation was observed at temperature of 28°C and higher. Pichia cultivation at 28°C or higher than 28°C enabled to obtain degraded rHA, despite the application of protease inhibitors or Casamino acid. **Fig. 4** shows analysis of elution fractions obtained during His₆-HA on Ni-NTA Agarose (12% SDS-PAGE, Coomassie stained) - for strains: SMD 1168, KM 71, and KM 71 with Casamino acid.

### Example 4. Enzymatic and MS/MS analysis of recombinant HA glycosylation

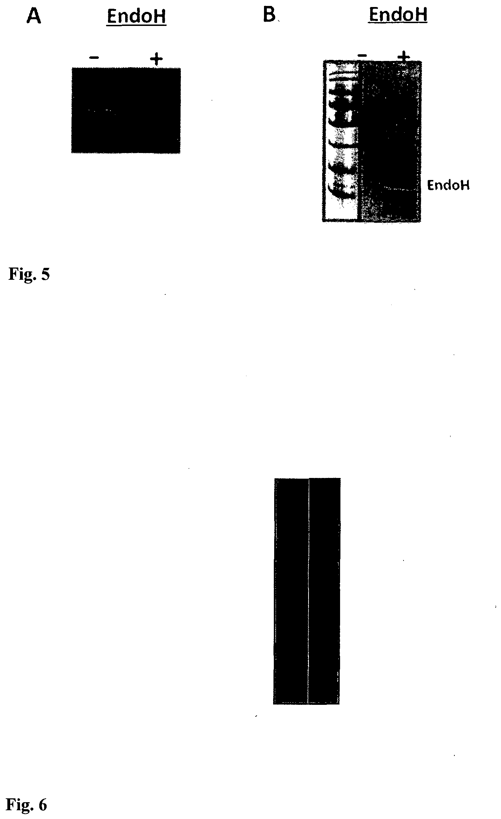

4,7 µg of rHA protein was incubated with 750 U of endoglycosidase H (New England BioLabs) at 37°C for 18 hours. Samples after the reaction were analysed by Western Blot and SDS-PAGE **(****Fig. 5****).** A band corresponding to deglycosylated rHA protein was cut off from the gel for MS/MS analysis.

The protein was digested by trypsin. The mixture of peptides (without reduction or alkylation) was applied onto RP-18 pre-column (Waters nanoAcquity 20 mm x 180 µm) with water and 0,1% trifluoroacetic acid and then onto HPLC RP-18 column (Waters nanoAcquity UPLC column 250 mm x 75 µm), using line gradient of acetonitrile (0-50% in 30 minutes) with 0,1% formic acid. Elution fraction from HPLC column was directed to MS ionization chamber (LTQ-FTICR, Thermo Electron). Protein Bank Data PDB (National Center of Biotechnology Information NCBI no. Version 20080624) and Mascot software (www.matrixscience.com) were used for the analysis of the resulting mass spectra. It is known that there are six potential N-glycosylation sites (5 in HA1 domain and 1 in HA2 domain) in HA sequence (Qinghai). Our research shows that all potential N-linked glycosylation sites undergo glycosylation. Subunit rHA1 N-glycosylation was confirmed with MS/MS of rHA, which was previously treated with EndoH.Only one glycosylation site in HA2 domain has been identified by MS/MS analysis.

### Example 5. Evaluation of rHA protein oligomerization

The level of rHA protein oligomerization has been evaluated. Native gel is shown in **Fig. 6****.**

### Example 6. Evaluation of rHA immunogenicity

Immunogenicity of rHA has been checked in mouse model. The experimental groups in the first trial consisted of 5 7-week-old Balb/c mice. The control group of 10 mice was administered adjuvant only. The animals were kept at a constant temperature of 22-24°C. The mice were immunized three times. Antigen dissolved in saline solution was administered at three doses: 1, 5, and 25 µg. Administration of 100 µl of vaccine was by intradermal injection (i.d.) in the walking pad of its hind paw. Sera samples were taken from all groups one week before the application of the first dose. There were three injections (first application of antigen and/or adjuvant + two booster shots) at an interval of three weeks between each dose in order to monitor immunological response. 25 µg of Sigma Adjuvant System was used in the first immunization. Two booster shots contained monophsphoryl lipid A and muramyl dipeptide at a dose of 25 µg each. Control mice received only a specific adjuvant. Blood samples were taken two weeks after each injection in order to determine the level of antibodies. Sera were stored at - 20°C.

The experimental groups in the second immunization experiment consisted of 7 7-week-old Balb/c mice. There were 5 mice in the control group. Antigen that dissolved in saline solution was administered only at a dose of 25 µg. However, two routes of administration were checked: intradermal injection (i.d.) in the walking pad of its hind paw and subcutaneous injection (s.c.) in the neck skin fold. Immunization schedule was the same as described above. The volume of the administered vaccine was 100 µl. 25 µg of Sigma Adjuvant System was applied in the first immunization. Two booster shots contained monophsphoryl lipid A and muramyl dipeptide at a dose of 25 µg each. Control group was only administered specific adjuvant. As in the first experiment, blood samples were taken two weeks after each injection in order to determine the level of antibodies. Sera were stored at -20°C.

In serum samples taken from the examined animals the level of antibody isotype IgG against influenza H5N1 haemagglutinin was determined. The level of antibodies was indicated with ELISA test. For the purposes of the test, MediSorp plates (NUNC) were coated with rH5 (17-530 aa, ΔRRRKKR, 6 x His) strain (A/swan/Poland/305-135V08/2006 (H5N1)) from baculovirus expression system (Oxford Expression Technologies) at 6µg/ml PBS concentration or filled with PBS (non-specific binding control). The plates were incubated at 2-8°C for the night, then 2% BSA was blocked in PBS for 1,5 h at 37°C. A series of mouse sera dilutions 1:2000 - 1:2000000 were prepared. Sera were diluted with 2% BSA/PBS. The plates with serum and blank samples - BLK (2%BSA/PBS) were incubated at 2-8°C for the night. The plate development included a 1-hour incubation at 37°C with labelled HRP antibodies acting against anti-mouse IgG Υ-chain specific (Sigma-Aldrich) antibodies, which were diluted 1:1000. The plates were washed in PBS containing Tween 20 at 0,05% concentration (PBST). 4 cycles of washing were done after coating, 2 after blocking, and 5 between the remaining phases of the procedures. Colour development of HRP was caused by TMB (Sigma). The plate was incubated with substrate for 30 minutes. Then, 0,5 M H₂SO₄ was added to inhibit HRP reaction. Absorbance was measured at λ=450nm. A₄₅₀ value which was obtained for BLK assay was subtracted from A₄₅₀ value read for serum assay. Results for non-coated rH5 plates acted as non-specific serum binding control (background). Serum assay was considered as H5 positive if their A₄₅₀ value was higher than average A₄₅₀ value for the control group increased by twice the standard deviation (*cut off*)*.* The endpoint titre was defined as the highest dilution of immunized chicken serum which would present absorbance value four times the average absorbance in control group.

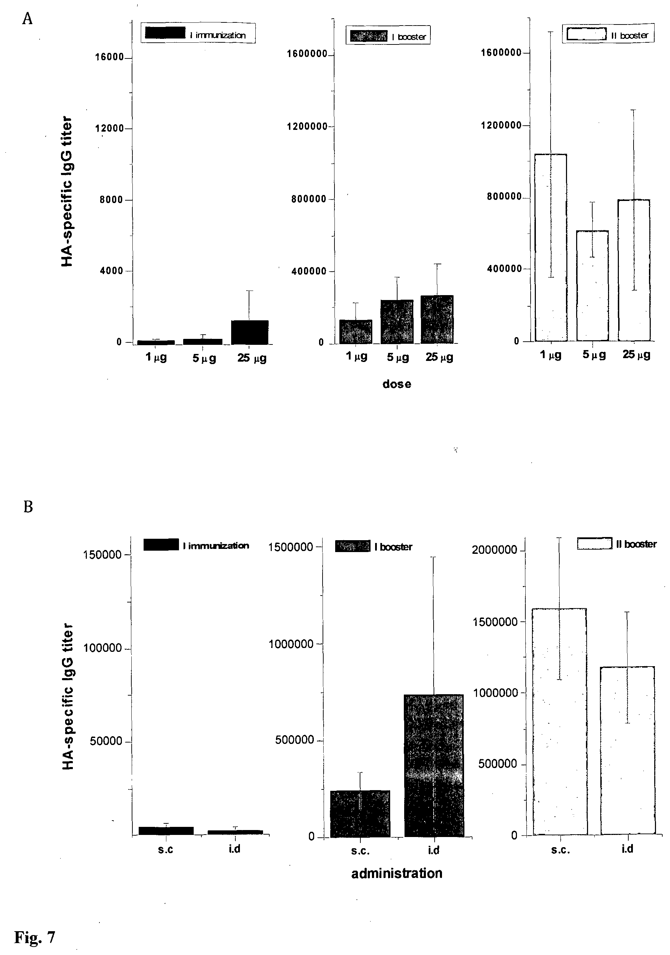

**Fig. 7** shows antibody IgG titer against H5 in mouse serum after administering rHA antigen obtained in Pichia pastoris cells at different doses of antigen and different routes of administration at 25 µg dose.

### Example 7. Immunogenicity of rHA antigens

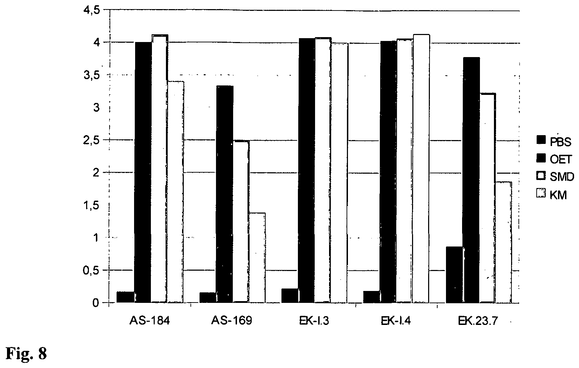

Immunogenicity of rH5 protein was determined with ELISA test. MediSorp (NUNC) plates were coated with three rH5 protein variants of the strain (A/swan/Poland/305-135V08/2006(H5N1)):
1. rH5 OET (17-530 aa, ΔRRRKKR, 6xHis) from baculovirus expression system (Oxford Expression technologies)
2. rH5 (17-531, 6xHis) from *P.pastoris* cells of KM71 strain
3. rH5 (17-531, 6xHis) from *P.pastoris* cells of SMD 1168 strain
   at 6 µg/ml PBS concentration or filled with PBS (non-specific binding control). The plates were incubated at 4°C for the night. Then, the plates were washed in PBST (PBS + 0,05% Tween 20) and blocked 2% BSA in PBS for 1,5 h at 37 °C. The experimental sera samples obtained from vaccinated chickens (AS-169, AS-184, EK-23.7) and mice (EK-1.3, EK-1.4) were diluted 1:200 with 2% BSA/PBSS (phosphate buffer containing 0,3 M NaCl +KCI). The plates with sera assays and control - PBS (2% BSA/PBS) were incubated at 2-8°C for the night. The plate development included a 1-hour incubation at 37°C with labelled HRP antibodies acting against anti-chicken IgG Υ-chain specific antibodies (assays AS-169, AS-184, EK-23.7) (Thermo Scientific) and labelled HRP antibodies acting against anti-mouse IgG Υ-chain specific antibodies (assays EK-1.3, EK-1.4) (Sigma-Aldrich). The plates were washed in PBS with Tween 20 at 0,05% concentration (PBST). Colour development of HRP was caused by TMB (Sigma). The plate was incubated with substrate for 30 minutes. After that, 0,5 M H₂SO₄ was added to inhibit HRP reaction. Absorbance was measured at λ=450nm, using microplate reader (Synergy 2; BioTek Instruments, USA) **[****fig. 8****].**

### Example 8. Cloning and overexpression of recombinant protein HA

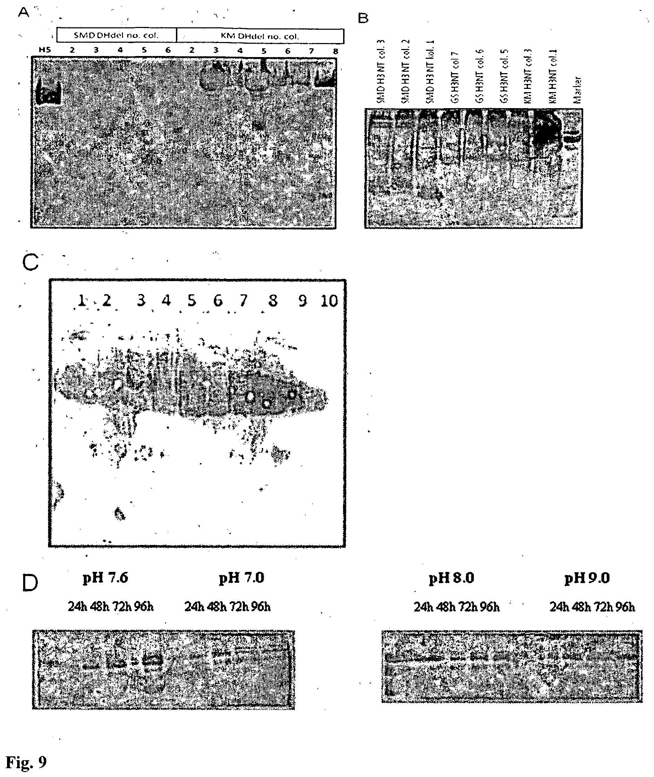

Plasmid pPICZαA (Invitrogen) and plasmid pJET/H1HA carrying H1N1 virus haemagglutinin gene (A/H1N1/Gdansk/036/2009 (H1N1)) have been used for construction of expression vector pPICZαA/H1DHHis₆. Plasmid pPICZaC (Invitrogen) and plasmid pGEM-Easy/H5HA carrying H5N1 virus haemagglutinin gene (A/swan/Poland/305-135V08/2006 (H5N1)) have been used for construction of expression vectors pPICZαC/H5DHdelHis₆ and pPICZαC/H5DHdel Plasmid pPICZαC (Invitrogen) and plasmid pMK-RQ/H3 carrying H3N2 virus haemagglutinin gene (Texas (H3N2)) have been used for construction of expression vectors pPICZαC/H3DH Plasmid pPICZαC (Invitrogen) and plasmid pMK-RQ/H7 carrying H7N9 virus haemagglutinin gene (Shanghai (H7N9)) have been used for construction of expression vectors pPICZαC/H7DH. Plasmid pPICZaC contains alcohol oxidase promoter 1 (*AOX1*) induced by methanol. The plasmid contains α factor sequence which comes from Saccharomyces cerevisiae, which ensures the secretion of recombinant protein to the medium. cDNA of 1569 bp, which codes hydrophilic domain (18-540 aa) of H1N1 virus haemagglutinin (A/H1N1/Gdansk/036/2009 (H1N1)), was amplified in PCR reaction with 5'TCCGTGGAATTCCATCATCATCATCATCATGACACATTATGTATAGGTTAT-3' and 3'TCAATACCGCGGTCAAATGAACTGGCGACAGTT-5' primers. The cleavage site (RRRKKR) in the hemagglutinin sequence of influenza virus H5N1 strain (A/swan/Poland/305-135V08/2006(H5Nl)) was deleted in a two step site-directed mutagenesis reaction using the primers:
5'GCCCTCAAGGAGAGAAAAAGAGAGGACTATTTGGAGCTATAGC-3' and 3'CGGGAGTTCCTCTCTTTTTCTCTCCTGATAAACCTCGATATCG-5' and 5'GCCCTCAAGGAGAGGGACTATTTGGAGCTATAGC-3' and 3'CGGGAGTTCCTCTCCCTGATAAACCTCGATATCG-5'. This resulted in a vector pGEM-Easy/H5HAdel carrying the hemagglutinin gene of influenza virus H5N1 strain (A/swan/Poland/305-135V08/2006 (H5N1)) with a deletion of the cleavage site (ΔRRRKKR). The cDNA (H5HAdel) of 1527 bp encoding the hydrophilic domain (aa 17-531, ΔRRRKKR) of H5N1 virus hemagglutinin (A/swan/Poland/305-135V08/2006 (H5N1)) was amplified in a two step PCR using the following primers: 5'GCACGATCGATGGATCAGATTTGCATTGGTTAC-3' and 3'AGTTATCCTTGGATGGTTGTAGTAGTAACTGGCGCCATAGCG-5'. In a second step following primers were used: 5'GCACGATCGATGGATCAGATTTGCATTGGTTAC-3' and 3'GTAGTAGTAACTGGCGCCATAGCG-5'. H5DHdel variant with no affinity tag was amplified by a one-step PCR reaction. The cDNA fragment (H3) encoding the hydrophilic domain of the hemagglutinin of influenza H3N2 virus was amplified by PCR using the following primers: 5' TCCGTGGGTACCGCAACGCTGTGCCTT-3' and 3'GCTAGCGGCCGCTCAATCTTTGTACCCTGA-5'. The cDNA fragment (H7) encoding the hydrophilic domain of the hemagglutinin of influenza H7N9 virus was amplified by PCR using the following primers: 5'TCCGTGGGTACCGACAAAATCTGCCTCGGA-3' and 3'GCTAGCGGCCGCTCAATCTTTGTAGCCGCT-5'. The sequence encoding His-Tag was added to cDNA sequence by the right primer development. PCR product was cloned into pPICZαA (H1) or pPICZαC (H5, H3 and H7) expression vector under the control of the AOX promoter. *Pichia pastoris* cells of strains KM 71 (his4, aox1::ARG4, arg4) and SMD 1168 (his4, pep4) (Invitrogen) were transformed with recombinant plasmids by electroporation. Integration of haemagglutinin gene into *P*. *pastoris* genome was confirmed by PCR. KM 71 and SMD 1168 transformant selection was performed using medium containing Zeocin (multiple passaging). A single colony of *P. pastoris* strain transformed with recombinant plasmid was cultured in BMGY medium (1% yeast extract, 2% peptone, 100 mM potassium phosphate, pH 6.0, 1.34% YNB 0.00004% biotin, 0.5% glycerol). The culture temerature was 28°C with shaking (at the frequency of 160 strokes per minute) until high cell density OD₆₀₀>3 is achieved. After centrifugation the cells were suspended in BMMY medium (1% yeast extract, 2% peptone, 100 mM potassium phosphate pH 7.8, 1.34% YNB, 0.00004% biotin, 5% methanol). In case of HIDHHis₆ induction in a broad range of pH was applied. The presence of recombinant protein both in medium and cells (control), was detected by SDS-PAGE and Western blotting. **Fig. 9** shows H1DHHis₆, H3DH, H5DHdelHis₆ and H5DH recombinant protein identified in culture supernatants of strains: KM71 and SMD1168.

### Example 9. Purification of recombinant HA protein

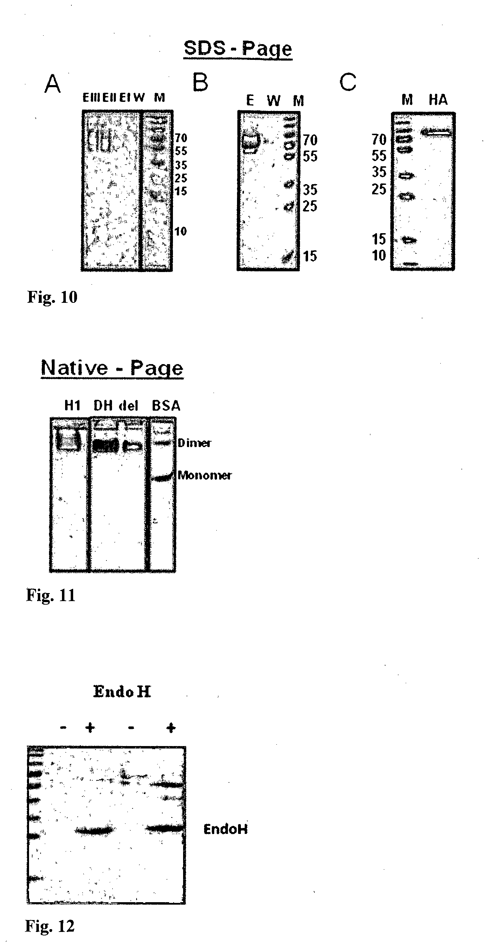

Recombinant haemagglutinins were purified from culture medium by affinity chromatography, using Ni-NTA Agarose resine (H1DHHis₆, H5DHdelHis₆). Protein binding to Ni-NTA Agarose was carried out in phosphate buffer solution (PBS) (pH 7.4 and 450 mM NaCl). The binding was carried out for 3 hours at 4°C, constantly shaking. The resine was rinsed with PBS pH 7.4 with 25 mM imidazole. HA proteins were eluted from the column with 250 mM imidazole in PBS pH 7.4. Recombinant antigens without the affinity tag were purified from the culture medium by ion exchange chromatography (elution with increasing salt concentration) and size exclusion chromatography. Specific amount of solution from each elution fraction was withdrawn for determining a protein level and SDS-PAGE analysis. The protein amount was determined spectrophotometrically using Bradford method by an absorbance measurement at 595 nm. The protein was then dialyzed against PBS pH 7.4. After dialysis the protein was liophylized and stored in temmperature -20°C. Analysis of elution fractions obtained during H1DHHis₆ and H5DHdelHis₆ purification on Ni-NTA Agarose and H5DHdel (ion exchange column) was shown in **Fig. 10****.**

### Example 10. Enzymatic and MS/MS analysis of recombinant HA glycosylation

5 µg of HIDHHis₆ protein was incubated with 750 U of endoglycosidase H (New England BioLabs) at 37°C for 18 hours. Samples after the reaction were analysed by Western Blotand SDS-PAGE **(****Fig. 12****).** A band corresponding to deglycosylated rHA protein was cut off from the gel for MS/MS analysis. The protein was digested by trypsin. The mixture of peptides (without reduction or alkylation) was applied onto RP-18 pre-column (Waters nanoAcquity 20mm x 180µm) with water and 0,1% trifluoroacetic acid and then onto HPLC RP-18 column (Waters nanoAcquity UPLC column 250 mm x 75 µm), using line gradient of acetonitrile (0-50% in 30 minutes) with 0,1% formic acid. Elution fraction from HPLC column was directed to MS ionization chamber (LTQ-FTICR, Thermo Electron). Protein Bank Data PDB (National Center of Biotechnology Information NCBI no. Version 20080624) and Mascot software (www.matrixscience.com) were used for the analysis of the resulting mass spectra. It is known that there are seven potential N-glycosylation sites (6 in HA1 domain and 1 in HA2 domain) in HA sequence (A/H1N1/Gdansk/036/2009 (H1N1)). Our research shows that N39, N292, N303 and N497 are glycosylated.

### Example 11. Evaluation of rHA protein oligomerization

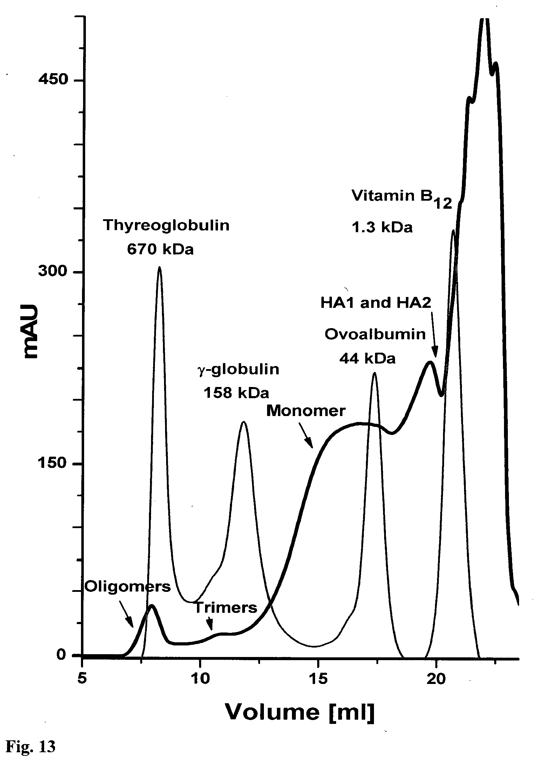

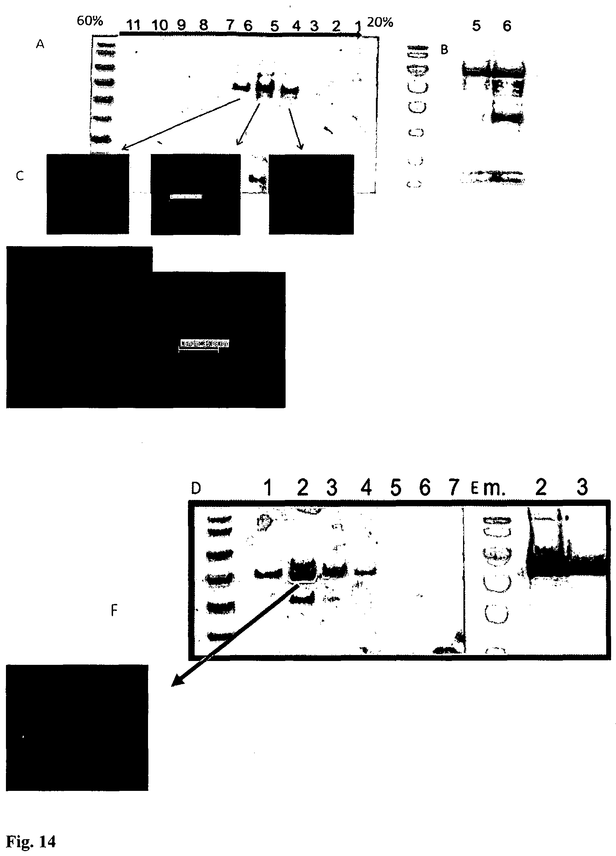

The level of rHA protein oligomerization has been evaluated using various techniques: electrophoresis in native conditions, size exclusion chromatography and transmission electron microscopy. Native gel with 10 µg of H1DHHis₆, and H5DHdelHis₆ is shown in **Fig. 11****.** 300 µg of H1DHHis₆ was analyzed on Superdex-200 column, pre-equilibrate with 10 mM Tris pH 7.8 and the protein elution was monitored at 280 nm **(****Fig. 13****).** Protein molecular weight marker standards (Bio-Rad) were used for column calibration and generation of standard curves to identify the molecular weights of the test protein samples. 300 µg of HIDHHis₆ and H5DHdelHis₆ of protein was separated in sucrose gradient (16 h, 27 000 rpm, 4°C). The individual fractions were analyzed by SDS-PAGE and Western blot. Selected fractions negatively stained with uranyl acetate were analyzed with transmission electron microscopy **(****Fig. 14****).**

### Example 12. Evaluation of rHA immunogenicity

Immunogenicity of rHA has been checked in mouse model. The experimental groups in the first trial consisted of 10 7-week-old Balb/c mice. The control group of 10 mice was administered adjuvant only. The animals were kept at a constant temperature of 22-24°C. The mice were immunized three times. Antigen dissolved in saline solution was administered at three doses: 1, 5, and 25 µg. Administration of 100 µl of vaccine was by intradermal injection (i.d.) in the walking pad of its hind paw. Sera samples were taken from all groups one week before the application of the first dose. There were three injections (first application of antigen and/or adjuvant + two booster shots) at an interval of three weeks between each dose in order to monitor immunological response. 25 µg of Sigma Adjuvant System was used in the first immunization. Two booster shots contained monophsphoryl lipid A and muramyl dipeptide at a dose of 25 µg each. Control mice received only a specific adjuvant. Blood samples were taken two weeks after each injection in order to determine the level of antibodies. Sera were stored at - 20°C. The experimental groups in the second immunization experiment consisted of 8 7-week-old Balb/c mice. There were 5 mice in the control group. In this experiment different route of administration of the antigen was tested - subcutaneously and other type of adjuvant - aluminum hydroxide. Moreover, additional dose of rH1DHHis₆ antigen - 0.5 µg was used. In addition, 8 mice were vaccinated with commercial vaccine containing seasonal H1 strain (California). The recombinant antigen suspended in saline solution supplemented with Alhydrogel (aluminum hydroxide) was administered subcutaneously (sc) into the neck skin fold. Commercial vaccine at a dose of 5 µg of H1 protein (total HA protein in one vaccine dose was 15 µg) were given without any adjuvant. Immunization schedule was the same as described above. The volume of the administered vaccine was 100 µl. Control group was only administered specific adjuvant. As in the first experiment, blood samples were taken two weeks after each injection in order to determine the level of antibodies. Sera were stored at -20°C.

Collected sera were assayed for antibodies against H1 HA by an ELISA method, using MaxiSorp plates (Nunc, Denmark) coated with mammalian cell-expressed HA (19-529 aa, 6xHis-tag at C-terminus) of A/South Carolina/1/18 (H1N1) strain of influenza A virus (Immune Technology, USA). Secondary labeling and its detection were done with reagents purchased from Sigma-Aldrich (USA). Sera samples from mice immunized with H1 protein were tested in parallel with sera from sham-immunized mice (negative controls). Mouse monoclonal anti H1 HA (A/New Jersey/8/1976(H1N1)) IgG1 antibodies (Immune Technology, USA) were used as positive controls. Sera samples, taken from individual groups at each time point of experiment were pooled, serially diluted in 2% BSA/PBS and applied onto the plates coated with H1 HA at 1.6 µg/ml in PBS (overnight, 2-8°C) and blocked with 2% BSA/PBS (1.5 h, 37°C). The tested samples were then incubated overnight at 2-8°C together with positive controls and blanks (sample diluent). Bound antibodies were subsequently detected with goat generated and horseradish peroxidase (HRP)-labeled antibodies against mouse IgG (γ-chain specific) at 1:1000 dilution in 2% BSA/PBS (1h, 37°C). TMB was used as a HRP substrate. After incubation for 30 min at room temperature, the reaction was stopped by the addition of 0.5 M sulfuric acid. The absorbance was measured at 450 nm with a microplate reader (Synergy 2; BioTek Instruments, USA).

Endpoint titer was defined as the highest dilution producing an A₄₅₀ value 4-fold higher than the mean A₄₅₀ value of the control group.

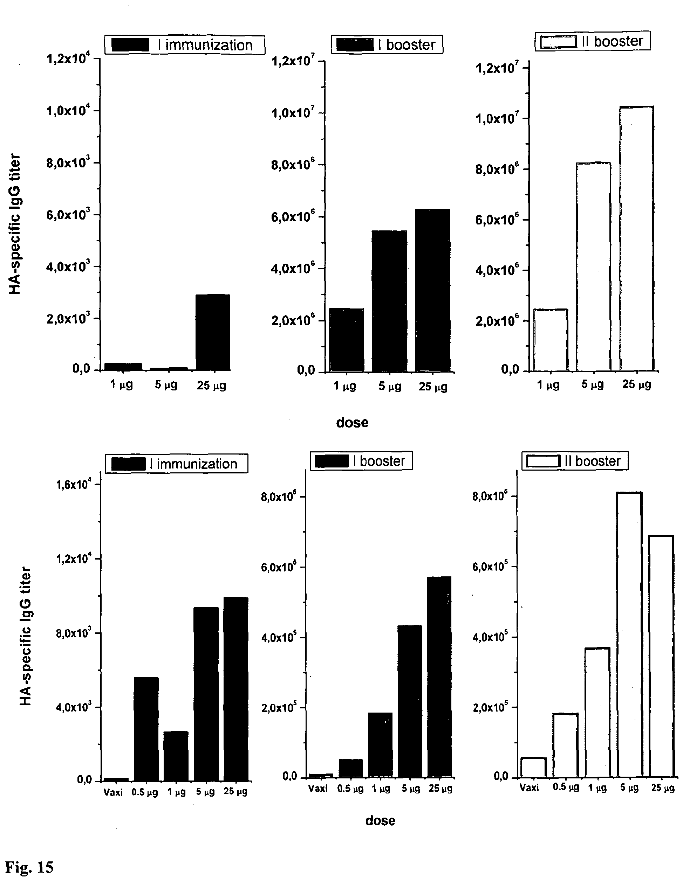

**Fig. 15** shows HA specific serum IgG endpoint titers after mice immunization with *P. pastoris* rH1 DHHis₆.

### Example 13. Specific immune response in chicken

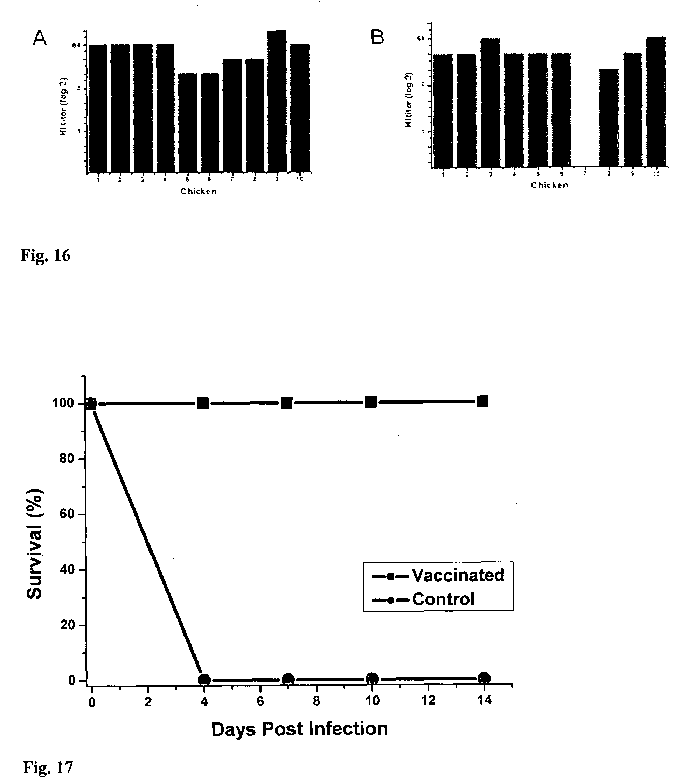

3-week-old layers were immunized with 25 µg of H5DHdelHis₆ protein (17-531, ΔRRRKKR). The recombinant antigen suspended in saline solution supplemented with Alhydrogel (aluminum hydroxide) was administered subcutaneously (sc) into the neck skin fold. The booster was done 4 weeks later. Control group was only administered specific adjuvant according to the same schedule. Blood samples were taken four weeks after immunization and two and four weeks after booster in order to determine the level of antibodies. Sera were stored at -20°C. 7-day-old broilers were immunized with 25 µg of H5DHdel protein (17-531, ΔRRRKKR). The recombinant antigen suspended in saline solution supplemented with Alhydrogel (aluminum hydroxide) was administered subcutaneously (sc) into the neck skin fold. The booster was done 3 weeks later. Control group was only administered specific adjuvant according to the same schedule. Blood samples were taken three weeks after each injection in order to determine the level of antibodies. Sera were stored at -20°C. **Fig. 16** shows the serum HI titer after booster against homologous H5N1 strain.

### Example 14. Protection of chicken from lethal H5N1 virus challenge.

3-week-old SPF chicken were immunized with 25 µg of H5DHdelHis₆ protein (17-531, ΔRRRKKR). The recombinant antigen suspended in saline solution supplemented with Alhydrogel (aluminum hydroxide) was administered subcutaneously (sc) into the neck skin fold. The booster was done 4 weeks later. Control group was only administered specific adjuvant according to the same schedule. Three weeks after second immunization experimental chicken and the control group were infected i.n./i.o with H5N1/turkey/35/07 virus at 10⁶ EID₅₀, The survival rate of SPF chicken immunized twice with H5DHdelHis₆ protein (black line) and not-immunized SPF chicken (control group, red line) is shown in **Fig. 17****.**

## Claims

1. A method for producing a secreted ectodomain of influenza virus haemagglutinin (HA) without signal peptide, **characterized by** the following steps:
a) inserting a sequence encoding the ectodomain of influenza H1 or H5, into a yeast expression vector comprising a signal sequence to direct said protein to the yeast secretory pathway, under control of the alcohol oxidase 1 (AOX1) promoter;
b) introducing said expression vector into Pichia pastoris cells of KM71 strain by electroporation;
c) culturing a Pichia pastoris transformant colony of KM71 strain obtained in step b), wherein the AOX1 promoter is induced with methanol; and
d) isolating the ectodomain of influenza HA without signal peptide from the culture medium of the transformant colony of step c).

2. The method of claims 1 wherein the secreted ectodomain of HA is a glycosylated protein having a trimeric or oligomeric form.

3. The method of claims 1 to 2 wherein the secreted ectodomain of HA protein comprises a deletion of a cleavage site, wherein 6 amino acids between HA1 and HA2 are removed.

4. The method of claims 1 to 3 wherein the secreted ectodomain of HA is derived from H5 influenza.

5. The method of claims 1 to 4, wherein the secreted ectodomain of HA further comprises an affinity tag.

6. A method for producing an influenza virus vaccine comprising a secreted ectodomain of influenza virus HA without signal peptide **characterized by**
a) obtaining a secreted ectodomain of influenza virus HA without signal peptide as defined in claims 1 to 5, using the method of claim 1; and
b) manufacturing a vaccine comprising said secreted ectodomain of influenza virus HA without signal peptide.

## Patentansprüche

1. Verfahren zur Herstellung einer sekretierten Ektodomäne des Influenzavirus-Hämagglutinin (HA) ohne Signalpeptid, **gekennzeichnet durch** die folgenden Schritte:
a) Einfügen einer Sequenz, die für die Ektodomäne von Influenza H1 oder H5 kodiert, in einen Hefeexpressionsvektor, der eine Signalsequenz umfasst, um das Protein, unter Kontrolle des Alkoholoxidase 1 (AOX1)-Promotors, auf den Hefesekretionsweg zu lenken;
b) Einführen des Expressionsvektors in Pichia pastoris-Zellen des KM71-Stammes durch Elektroporation;
c) Kultivieren einer transformierten Pichia pastoris-Kolonie des KM71-Stammes, die in Schritt a) erhalten wurde, wobei der AOX1-Promotor mit Methanol induziert wird; und
d) Isolieren der Ektodomäne des Influenza-HA ohne Signalpeptid aus dem Kulturmedium der transformierten Kolonie von Schritt c).

2. Verfahren nach Anspruch 1, wobei die sekretierte Ektodomäne von HA ein glykosyliertes Protein mit einer trimeren oder oligomeren Form ist.

3. Verfahren nach den Ansprüchen 1 bis 2, wobei die sekretierte Ektodomäne des HA-Proteins eine Deletion einer Spaltstelle umfasst, wobei 6 Aminosäuren zwischen HA1 und HA2 entfernt sind.

4. Verfahren nach den Ansprüchen 1 bis 3, wobei die sekretierte Ektodomäne von HA von H5-Influenza abgeleitet ist.

5. Verfahren nach den Ansprüchen 1 bis 4, wobei die sekretierte Ektodomäne von HA ferner eine Affinitäts-Tag umfasst.

6. Verfahren zur Herstellung eines Influenzavirus-Impfstoffs, die eine sekretierte Ektodomäne des Influenzavirus-HA ohne Signalpeptid umfasst, **gekennzeichnet durch**
a) Erhalten einer sekretierten Ektodomäne des Influenzavirus-HA ohne Signalpeptid, wie in den Ansprüchen 1 bis 5 definiert ist, unter Verwendung des Verfahrens von Anspruch 1; und
b) Herstellen eines Impfstoffs, der die sekretierte Ektodomäne des Influenzavirus-HA ohne Signalpeptid umfasst.

## Revendications

1. Procédé de production d'un ectodomaine sécrété d'hémagglutinine (HA) du virus de la grippe sans peptide signal, **caractérisé par** les étapes suivantes :
a) l'insertion d'une séquence codant pour l'ectodomaine de la grippe H1 ou H5, en un vecteur d'expression de levure comprenant une séquence signal pour diriger ladite protéine vers la voie de sécrétion de levure, sous le contrôle du promoteur d'alcool oxydase 1 (AOX1) ;
b) introduction dudit vecteur d'expression dans des cellules de Pichia pastoris de souche KM71 par électroporation ;
c) culture d'une colonie transformante de *Pichia pastoris* de souche KM71 obtenue à l'étape b), dans laquelle le promoteur AOX1 est induit avec du méthanol ; et
d) isolation de l'ectodomaine de la grippe HA sans peptide signal du milieu de la culture de la colonie transformante de l'étape c).

2. Procédé selon la revendication 1, dans lequel l'ectodomaine sécrété d'HA est une protéine glycosylée ayant une forme trimérique ou oligomérique.

3. Procédé selon les revendications 1 à 2, dans lequel l'ectodomaine sécrété de la protéine HA comprend une délétion d'un site de clivage, dans lequel 6 acides aminés entre HA1 et HA2 sont enlevés.

4. Procédé selon les revendications 1 à 3, dans lequel l'ectodomaine sécrété d'HA est dérivé de la grippe H5.

5. Procédé selon les revendications 1 à 4, dans lequel l'ectodomaine sécrété d'HA comprend en outre une étiquette d'affinité.

6. Procédé de production d'un vaccin contre le virus de la grippe comprenant un ectodomaine sécrété du virus de la grippe HA sans peptide signal **caractérisé par**
a) l'obtention d'un ectodomaine sécrété du virus de la grippe HA sans peptide signal tel que défini dans les revendications 1 à 5, en utilisant le procédé de la revendication 1 ; et
b) la fabrication d'un vaccin comprenant ledit ectodomaine sécrété du virus de la grippe HA sans peptide signal.
